# DEMANDE DE BREVET EUROPEEN

(11) **EP 1 473 020 A1**
(43) Date de publication de la demande: **03.11.2004**
(21) Numéro de dépôt: 04291078.6
(22) Date de dépôt: 26.04.2004
(51) Int. Cl.: A61K 7/06, A61K 7/50

(54) **Composition détergente contenant du nitrure de bore associé à au moins une huile**

(30) Priorité: 28.04.2003 FR 0305183
(71) Demandeur: L'OREAL, 75008 Paris (FR)
(72) Inventeur: Parris, Eric, 92600 Asnieres (FR); Gawtrey, Jonathan, 92100 Boulogne (FR)
(74) Mandataire: Casalonga, Axel

(57) **Abrégé**

La présente invention décrit une composition détergente comprenant, dans un milieu aqueux cosmétiquement acceptable du nitrure de bore associé à au moins une huile, et au moins un tensioactif détergent anionique.

Cette composition peut être utilisée comme shampoing et permet d'obtenir de bonnes propriétés de conditionnement des cheveux.

## Description

La présente invention concerne une composition détergente contenant du nitrure de bore associé à au moins une huile, et un procédé de traitement capillaire mettant en oeuvre cette composition.

Dans le domaine de la cosmétique, on cherche notamment à améliorer le conditionnement des cheveux. Par conditionnement, on entend des propriétés de démêlage facile, de brillance, de douceur au toucher et de glissant.

L'utilisation de particules de nitrure de bore est connue en cosmétique, et plus particulièrement dans le domaine du maquillage ou dans des compositions de protection contre le rayonnement ultraviolet comme cela est décrit dans les demandes de brevet DE 199 23 773 et DE 199 23 667.

La demande EP 1 077 058 décrit des compositions cosmétiques ou dermatologiques, de type eau-dans-huile, sans émulsifiant comprenant du nitrure de bore qui peut être enrobé avec un polydiméthylsiloxane, dans des compositions de soin de la peau.

La Demanderesse a trouvé de manière surprenante qu'en utilisant, dans des compositions de traitement capillaire, des particules de nitrure de bore en association avec au moins une huile, il était possible d'obtenir un très bon effet de conditionnement des cheveux.

Sans être tenu par une quelconque théorie, il semblerait que dans ces conditions, on augmente significativement le dépôt de nitrure de bore sur les cheveux, d'où une efficacité accrue.

Par ailleurs, cet effet de conditionnement peut être rémanent au rinçage et au passage du peigne ou d'une brosse à cheveux.

L'invention a donc pour objet une composition détergente comprenant, dans un milieu aqueux cosmétiquement acceptable, du nitrure de bore associé à au moins une huile.

Un objet supplémentaire de la présente invention est un procédé de traitement des matières kératiniques mettant en oeuvre une composition selon l'invention.

D'autres objets, caractéristiques, aspects et avantages de l'invention apparaîtront encore plus clairement à la lecture de la description et des divers exemples qui suivent.

Selon l'invention, la composition détergente comprend du nitrure de bore associé à au moins une huile, et au moins un tensioactif détergent anionique.

Le nitrure de bore utilisé dans la présente invention peut être enrobé par cette huile ou ces huiles. L'enrobage est effectué, de préférence, avant introduction dans la composition détergente via toutes les technologies classiques telles que, par exemple, extrusion ou addition d'un tiers solvant volatil.

Le rapport pondéral nitrure de bore/huile(s) est de préférence supérieur ou égal à 50/50, mieux encore supérieur ou égal à 60/40, plus préférentiellement compris entre 60/40 et 90/10 et encore plus préférentiellement compris entre 80/20 et 90/10.

Le nitrure de bore présente de préférence une taille primaire moyenne en nombre comprise entre 1 et 50 *µ*m, mieux encore entre 5 et 30 *µ*m, plus préférentiellement entre 5 et 25 *µ*m, et encore plus préférentiellement entre 7 et 15 *µ*m.

Au sens de la présente invention, on entend par "taille primaire de particule", la dimension maximale qu'il est possible de mesurer entre deux points diamétralement opposés d'une particule individuelle. La taille peut être déterminée, par exemple, par microscopie électronique à transmission ou à partir de la mesure de la surface spécifique par la méthode BET ou bien par l'intermédiaire d'un granulomètre laser.

Les particules selon l'invention peuvent, avoir une forme quelconque, par exemple, de préférence la forme de lamelles, de paillettes, d'aiguilles, de plaquettes ou des formes totalement aléatoires.

Le nitrure de bore tel que défini ci-dessus est de préférence présent en une quantité comprise entre 0,1 et 35 % en poids, mieux encore en une quantité comprise entre 1 et 30 % en poids, et encore plus préférentiellement en une quantité comprise entre 5 et 25 % en poids par rapport au poids de la composition.

Par huile, on entend au sens de la présente invention, une substance liquide à température ambiante, par exemple de 25 °C, et sous pression atmosphérique, et insoluble dans l'eau.

Par insoluble dans l'eau, on entend au sens de la présente invention, une substance qui présente une solubilité dan l'eau pure inférieure à 1 % à 25 °C et sous pression atmosphérique.

Les huiles utilisées dans la présente invention présente une viscosité dynamique à 25 °C, inférieure à 1 Pa.s (1000 cps), de préférence comprise entre 10⁻³ et 0,1 Pa.s (1 et 100 cps). La viscosité dynamique est mesurée à 25 °C avec un taux de cisaillement de 100 s⁻¹, par exemple, avec l'appareil référencé RM 180 Rheomat de la société METTLER.

Les huiles pouvant être utilisées dans la présente invention sont choisies de préférence, parmi les huiles végétales, les huiles minérales, les huiles de synthèse, les huiles siliconées et les esters d'acide gras.

Parmi les huiles végétales pouvant être utilisées dans la présente invention, on peut notamment citer l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot et l'huile de calophyllum.

Des exemples d'huiles minérales sont l'huile de paraffine et l'huile de vaseline.

Les huiles de synthèse peuvent notamment être choisies parmi les polydécènes, le squalane, les poly(α-oléfines) comme l'isododécane ou l'isohexadécane, les huiles végétales transestérifiées et les huiles fluorées.

On peut également utiliser des esters d'acide gras, tels que par exemple, les composés de formule RₐCOOR_{b} dans laquelle Rₐ représente le reste d'un acide gras supérieur comportant de 6 à 29 atomes de carbone et R_{b} représente une chaîne hydrocarbonée contenant de 3 à 30 atomes de carbone, telles que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle.

A titre d'exemples d'huiles siliconées, on peut notamment citer les polydiméthylsiloxanes (PDMS), les polyorganosiloxanes phénylés tels que les phényltriméthicones, les phényltriméthylsiloxydiphénylsiloxanes, les diphénylméthyldiméthyltrisiloxanes, les diphényldiméthicones, les phényldiméthicones, les polyméthyl-phénylsiloxanes, éventuellement fluorés ; les polysiloxanes modifiés par des acides gras, des alcools gras ou des polyoxyalkylènes, les silicones fluorées, les huiles siliconées perfluorées.

Parmi les huiles siliconées préférées, on peut citer les polydiméthylsiloxanes, les polyméthylphénylsiloxanes, les silicones comportant des séquences ou des greffons polyoxyalkylènes, en particulier polyoxyéthylène ou copoly(oxyéthylène/oxypropylène) telles que les diméthiconecopolyols, les silicones portant à la fois des groupes hydrophobes hydrocarbonés (par exemple des groupes alkyle en C₂-C₃₀) et des séquences ou greffons polyoxyéthylénés ou copoly(oxyéthylénés/oxypropylénés) telles que les alkyldiméthiconecopolyols, les silicones portant des groupes fluorés ou perfluorés telles que les polydiméthylsiloxanes perfluoroalkylés et les polyméthylphénylsiloxanes perfluoroalkylés, et leurs mélanges.

Ces huiles siliconées peuvent comporter éventuellement des groupes alkyle ou alcoxy en bout de chaîne siliconée ou pendante.

Comme huile siliconée utilisable dans l'invention, on peut encore citer les silicones linéaires ou cycliques, et comportant en particulier de 2 à 7 atomes de silicium. On peut notamment citer l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane et leurs mélanges.

Les huiles particulièrement préférées dans le cadre de la présente invention sont l'huile d'avocat, l'isohexadécane, une paraffine liquide et les polydiméthylsiloxanes.

La ou les huile(s) telle(s) que définie(s) ci-dessus est ou sont de préférence présente(s) en une quantité comprise entre 0,01 et 20 % en poids, encore plus préférentiellement en une quantité comprise entre 0,1 et 10 % en poids par rapport au poids de la composition.

A titre de tensioactifs détergents anioniques utilisables, seuls ou en mélanges, dans le cadre de la présente invention, on peut citer notamment les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les sulfates d'alkyle, les alkyléther-sulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les α-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 6 à 24 atomes de carbone et le groupe aryle désignant de préférence un groupe phényle ou benzyle. On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les sulfosuccinamates d'alkyle, les iséthionates d'acyle et les N-acyltaurates, le groupe alkyle ou acyle de tous ces composés comportant de 12 à 20 atomes de carbone. Parmi les tensioactifs anioniques encore utilisables, on peut également citer les lactylates d'acyle dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éthercarboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Parmi les tensioactifs anioniques cités ci-dessus, on préfère utiliser selon l'invention les sels, en particulier de sodium, de magnésium ou d'ammonium, de sulfates d'alkyle ; d'alkyléthersulfates, comme le lauryléthersulfate de sodium, de préférence à 2 ou 3 moles d'oxyde d'éthylène ; d'alkyléthercarboxylates ; et leurs mélanges, les groupes alkyle comportant généralement de 6 à 24 atomes de carbone, et de préférence de 8 à 16 atomes de carbone.

Les tensioactifs anioniques sont généralement présents en une quantité comprise entre 3 et 50 % en poids, de préférence entre 4 et 30 % en poids, encore plus préférentiellement entre 5 et 20 % en poids par rapport au poids total de la composition détergente.

La composition détergente selon l'invention peut comprendre en outre un ou plusieurs tensioactifs non ioniques, amphotères, et cationiques, et de préférence au moins un tensioactif amphotère ou non ionique, et encore plus préférentiellement au moins un tensioactif amphotère.

Les agents tensioactifs non-ioniques utilisables dans la composition selon l'invention, sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine ; et leurs mélanges.

Parmi les tensioactifs non-ioniques cités ci-dessus, on utilise de préférence les alkyl(C₆-C₂₄)polyglycosides.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant, au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL® , tels que décrits dans les brevets US-2 528 378 et US-2 781 354 et classés dans le dictionnaire CTFA, 3^{ème} édition, 1982, sous les dénominations Amphocarboxyglycinate et Amphocarboxypropionate de structures respectives (2) et (3) :

R₂-CONHCH₂CH₂-N⁺(R₃)(R₄)(CH₂COO⁻) (2)

dans laquelle :
R₂ représente un groupe alkyle dérivé d'un acide R₂-COOH présent dans l'huile de coprah hydrolysée, un groupe heptyle, nonyle
ou undécyle,

R₃ représente un groupe bêta-hydroxyéthyle, et

R₄ représente un groupe carboxyméthyle ; et

R_{2'}-CONHCH₂CH₂-N(B)(C) (3)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R₂, représente un groupe alkyle d'un acide R_{g} -COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL® C2M concentré par la société RHODIA.

Parmi les tensioactifs amphotères, on utilise de préférence les alkyl(C₈-C₂₀)bétaïnes, les alkyl(C₈-C₂₀)amidoalkyl(C₆-C₈)bétaïnes, les alkylamphodiacétates et leurs mélanges.

A titre de tensioactif cationique, on peut notamment citer cationiques les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.

Les tensioactifs cationiques utilisés de préférence dans la présente invention sont les sels d'alkylamidoalkyltrialkylammonium, les dérivés d'imidazoline, ou les oxydes d'amines à caractère cationique.

Les tensioactifs différents des tensioactifs anioniques sont généralement présents en une quantité comprise entre 0,5 et 25 % en poids, de préférence entre 0,5 et 15 % en poids, encore plus préférentiellement entre 1 et 10 % en poids par rapport au poids total de la composition détergente.

Par milieu cosmétiquement acceptable, on entend un milieu compatible avec les cheveux, mais aussi d'odeur, d'aspect et de toucher agréables.

Le milieu aqueux cosmétiquement acceptable comprend l'eau ou un mélange d'eau et d'un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

L'eau est présente de préférence en une quantité allant de 35 à 97 % en poids, mieux encore de 40 à 95 % en poids et encore plus préférentiellement de 45 à 90 % en poids par rapport au poids total de la composition.

La composition selon l'invention peut comprendre en outre un ou plusieurs additifs classiques bien connus dans la technique, tels que des polymères anioniques, cationiques, non-ioniques amphotères ou zwitteroniques ; des filtres UV ; des parfums ; des colorants ; des épaississants naturels ou synthétiques ; des alcools gras en C₁₂-C₃₀ ; des agents nacrants ; des conservateurs ; des agents de stabilisation de pH ; des vitamines, des provitamines ; des agents antibactériens ; des agents anti-oxydants ou réducteurs, et des agents acides ou alcalins.

La composition selon l'invention peut notamment comprendre en outre un ou plusieurs polymères cationiques. Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français n^{os} 2 505 348 et 2 542 997. Parmi ces polymères, on peut citer :
(1) les homopolymères ou copolymères dérivés d'esters ou d'amides de l'acide acrylique ou méthacrylique ;
(2) les dérivés d'éthers de cellulose comportant des groupements ammonium quaternaire décrits dans le brevet français 1 492 597 ;
(3) les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium ou de diméthyldiallylammonium ;
(4) les polysaccharides cationiques décrits plus particulièrement dans les brevets US 3 589 578 et 4 031 307 tels que les gommes de guar contenant des groupements cationiques trialkylammonium ;
(5) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361 ;
(6) les polyaminoamides solubles dans l'eau, tels que ceux notamment décrits dans les brevets français 2 252 840 et 2 368 508 ;
(7) les dérivés de polyaminoamides, par exemple, les polymères acide adipique/diakylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
(8) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347 ;
(9) les cyclopolymères d'alkyldiallylamine ou de dialkyldiallylammonium tels que l'homopolymère de chlorure de diméthyldiallyl-ammonium et les copolymères de chlorure de diallyldiméthylammonium et d'acrylamide ;
(10) les polymères de diammonium quaternaire présentant une masse moléculaire moyenne en nombre généralement comprise entre 1000 et 100000, tels que, ceux décrits, par exemple, dans les brevets français 2 320 330, 2 270 846, 2 316 271, 2 336 434 et 2 413 907 et les brevets US 2 273 780, 2 375 853, 2 388 614, 2 454 547, 3 206.462, 2 261 002, 2 271 378, 3.874.870, 4.001.432, 3 929 990, 3 966 904, 4 005 193, 4 025 617, 4 025 627, 4 025 653, 4 026 945 et 4 027 020 ;
(11) les polymères de polyammonium quaternaire tels que ceux notamment décrits dans la demande de brevet EP-A-122 324 ;
(12) les polymères quaternaires de vinylpyrrolidone et de vinylimidazole tels que, par exemple, les produits commercialisés sous les dénominations Luviquat® FC 905, FC 550 et FC 370 par la société B.A.S.F ;
(13) les polyamines comme le Polyquart® H vendu par HENKEL, référencées sous le nom de « POLYETHYLENEGLYCOL (15) TALLOW POLYAMINE » dans le dictionnaire CTFA ;
(14) les polymères réticulés de sels de méthacryloyloxyalkyl(C₁-C₄) trialkyl(C₁-C₄)ammonium tels que ceux commercialisés sous le nom de SALCARE® SC 92, SALCARE® SC 95 et SALCARE® SC 96 par la Société ALLIED COLLOIDS ; et leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on peut utiliser de préférence les dérivés d'éther de cellulose quaternaires, les gommes de guar cationiques, les cyclopolymères cationiques, les polymères quaternaires de vinylpyrrolidone et de vinylimidazole, et leurs mélanges.

L'homme de métier veillera à choisir les éventuels additifs et leur quantité de manière à ce qu'ils ne nuisent pas aux propriétés des compositions de la présente invention.

De préférence, la composition de l'invention est liquide et encore plus préférentiellement se présente sous la forme d'une émulsion huile-dans-eau.

Les compositions conformes à l'invention peuvent être utilisées en tant que shampoings.

Un autre objet de l'invention est un procédé de traitement capillaire des cheveux qui consiste à appliquer une quantité efficace d'une composition telle que décrite ci-dessus, sur les cheveux, à rincer après un éventuel temps de pose.

Les exemples suivants illustrent la présente invention.

### EXEMPLES

### Exemple 1

On prépare du nitrure de bore enrobé à partir des composants indiqués dans le tableau ci-dessous dans les rapports indiqués ci-dessous :

| Huile d'enrobage | Nitrure de bore | Rapport pondéral Nitrure de bore/huile |
|---|---|---|
| Huile d'avocat | Poudre de Nitrure de bore⁽⁴⁾, de taille moyenne des particules de 9-11 *µ*m | 80/20 |
| | | 90/10 |
| Isohexadécane⁽¹⁾ | | 80/20 |
| | | 75/25 |
| | | 65/35 |
| Diméthicone⁽²⁾ | | 80/20 |
| | | 75/25 |
| | | 65/35 |
| Paraffine liquide⁽³⁾ | | 80/20 |

| | | |
|---|---|---|
| ⁽¹⁾ vendu sous la dénomination commerciale Permethyl 101A par la société Bayer | | |
| ⁽²⁾ vendu sous la dénomination commerciale DC 200 Fluid par la société Dow Corning - 5 mm²/s | | |
| ⁽³⁾ vendu sous la dénomination commerciale Marcol 82 par la société ESSO - 15 mm²/s | | |
| ⁽⁴⁾ vendu sous la dénomination commerciale CC6004 par la société Advanced Ceramics | | |

On a pesé 20 g d'huile d'enrobage telle qu'indiquée dans le tableau ci-dessus et on a ajouté 20 g d'un solvant volatil de l'huile (un alcool absolu, par exemple). On a homogénéisé la solution par agitation mécanique, par exemple au moyen d'un barreau aimanté ou d'une pale.

On a mélangé ensuite la solution obtenue avec 80 g de nitrure de bore sous agitation permanente pour bien homogénéiser le tout.

On a maintenu l'agitation jusqu'à disparition complète du solvant volatil.

### Exemples 2 à 6

Différents shampoings sont préparés avec le nitrure de bore enrobé. Les proportions sont indiquées en % en poids.

| Composition | Ex 2 |
|---|---|
| Lauryléthersulfate de sodium à 70 % | 12 (MA) |
| Cocobétaïne à 30 % | 2,2 (MA) |
| Cocamide MIPA⁽¹⁾ | 1,5 (MA) |
| Carbomer⁽²⁾ | 0,2 |
| Diméthicone⁽³⁾ | 2,7 |
| Niture de bore enrobé avec l'isohexadécane, comme décrit dans l'exemple 1. | 20 |
| (Rapport Nitrure de bore/huile) | (75/25) |
| Conservateur qs | |
| Eau qsp | 100 |

| | |
|---|---|
| MA : Matière Active ⁽¹⁾ vendu sous la marque commerciale Empilan CIS par la société HUNSTSMAN | |
| ⁽²⁾ vendu sous la marque commerciale Carbopol 980 par la société Noveon | |
| ⁽³⁾ vendu sous la marque commerciale DC200 FLUID 60 000 mm²/s par la société DOW CORNING | |

| Composition | Ex. 3 | Ex. 4 | Ex. 5 | Ex. 6 |
|---|---|---|---|---|
| Lauryléthersulfate de sodium à 70 % | 12 (MA) | 12 (MA) | 16 (MA) | 5 (MA) |
| Cocobétaïne à 30 % | 4 (MA) | 2,4 (MA) | 5 (MA) | - |
| Acide laureth-5 carboxylique à 90% | - | - | 3 (MA) | - |
| Décyl glucoside à 40% | - | - | - | 10 (MA) |
| Chlorure de sodium | 8 (MA) | - | - | - |
| Cocamide MIPA | - | 1,5 (MA) | 2,5 (MA) | - |
| Carbomer | - | 0,2 | - | - |
| Isohexadécane | - | - | 2 (MA) | - |
| Copolymère acrylate d'hydroxyéthyle/acryloyldiméthyltaurate de sodium à 40% | - | - | - | 1,6 (MA) |
| Polyquaternium-6 | - | 0,2 (MA) | - | - |
| Polyquaternium-10 | 0,3 (MA) | - | - | - |
| Niture de bore enrobé avec la diméthicone, comme décrit dans l'exemple 1. | 20 | 20 | 20 | 20 |
| (Rapport Nitrure de bore/huile) | (65/35) | (65/35) | (70/30) | (70/30) |
| Conservateur qs | | | | |
| Eau qsp | 100 | 100 | 100 | 100 |
| MA : Matière Active | | | | |

Ces compositions appliquées sur cheveux naturels leur confèrent après 3 minutes de temps de pose et un rinçage, un excellent effet conditionneur.

## Revendications

1. Composition détergente, comprenant dans un milieu aqueux cosmétiquement acceptable, du nitrure de bore associé à au moins une huile et au moins un tensioactif détergent anionique.

2. Composition selon la revendication 1, **caractérisée en ce que** le nitrure de bore est enrobé par au moins une huile.

3. Composition selon la revendication 1 ou 2, **caractérisée en ce que** le rapport pondéral nitrure de bore/huile(s) est supérieur ou égal à 50/50.

4. Composition selon la revendication 3, **caractérisée en ce que** le rapport pondéral nitrure de bore/huile(s) est supérieur ou égal à 60/40, de préférence compris entre 60/40 et 90/10.

5. Composition selon l'une quelconque des revendications 1 à 4, **caractérisée en ce que** le nitrure de bore présente une taille primaire moyenne en nombre comprise entre 1 et 50 *µ*m, de préférence comprise entre 5 et 30 *µ*m.

6. Composition selon l'une quelconque des revendications précédentes, **caractérisée en ce que** le nitrure de bore est présent en une quantité comprise entre 0,1 et 35 % en poids, de préférence entre 1 et 30 % en poids par rapport au poids total de la composition.

7. Composition selon l'une quelconque des revendications précédentes, **caractérisée en ce que** l'huile présente une viscosité dynamique comprise inférieure à 1 Pa.s à 25 °C.

8. Composition selon la revendication 7, **caractérisée en ce que** l'huile présente une viscosité dynamique comprise entre 10⁻³ et 0,1 Pa.s à 25 °C.

9. Composition selon l'une quelconque des revendications précédentes, **caractérisée en ce que** l'huile est choisie parmi les huiles végétales, les huiles minérales, les huiles de synthèse, les huiles siliconées et les esters d'acides gras.

10. Composition selon la revendication 9, **caractérisée en ce que** l'huile est choisie parmi l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; l'huile de paraffine et l'huile de vaseline ; les polydécènes, le squalane, les poly(α-oléfines) comme l'isododécane ou l'isohexadécane, les huiles végétales transestérifiées et les huiles fluorées ; les composés de formule RₐCOOR_{b} dans laquelle Rₐ représente le reste d'un acide gras supérieur comportant de 6 à 29 atomes de carbone et R_{b} représente une chaîne hydrocarbonée contenant de 3 à 30 atomes de carbone, les polydiméthylsiloxanes (PDMS), les polyorganosiloxanes phénylés ; les polysiloxanes modifiés par des acides gras, des alcools gras ou des polyoxyalkylènes, les silicones fluorées, les huiles siliconées perfluorées ; les silicones linéaires ou cycliques comportant de 2 à 7 atomes de silicium.

11. Composition selon la revendication 10, **caractérisée en ce que** l'huile est choisie parmi l'huile d'avocat, l'isohexadécane, une paraffine liquide et les polydiméthylsiloxanes.

12. Composition selon l'une quelconque des revendications précédentes, **caractérisée en ce que** la ou le(s) huile(s) est ou sont présente(s) en une quantité comprise entre 0,01 et 20 % en poids, de préférence entre 0,1 et 10 % en poids par rapport au poids total de la composition.

13. Composition selon l'une quelconque des revendications précédentes, **caractérisée en ce que** les tensioactifs anioniques sont choisis parmi les sels de métaux alcalins, les sels d'ammonium, les sels d'amines, les sels d'aminoalcools ou les sels de métaux alcalino-terreux, des composés suivants : les sulfates d'alkyle, les alkyléther-sulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les α-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 6 à 24 atomes de carbone et le groupe aryle désignant de préférence un groupe phényle ou benzyle ; les esters d'alkyle en C₆-C₂₄ et d'acides polyglycoside-carboxyliques ; les sulfosuccinamates d'alkyle, les iséthionates d'acyle et les N-acyltaurates, le groupe alkyle ou acyle de tous ces composés comportant de 12 à 20 atomes de carbone.

14. Composition selon la revendication 13, **caractérisée en ce que** les tensioactifs anioniques sont choisis parmi les sels de sulfates d'alkyle, d'alkyléther-sulfates, de préférence à 2 ou 3 moles d'oxyde d'éthylène, et d'alkyléthercarboxylates, les groupes alkyle comportant de 6 à 24 atomes de carbone.

15. Composition selon l'une quelconque des revendications précédentes, **caractérisée en ce que** les tensioactifs anioniques sont présents en une quantité comprise entre 3 et 50 % en poids, de préférence entre 4 et 30 % en poids par rapport au poids total de la composition.

16. Composition selon l'une quelconque des revendications précédentes, **caractérisée en ce qu'**elle comprend en outre un ou plusieurs tensioactifs non ioniques, amphotères ou cationiques.

17. Composition selon l'une quelconque des revendications précédentes, **caractérisée en ce qu'**elle comprend en outre au moins un tensioactif amphotère ou non ionique.

18. Composition selon la revendication 16 ou 17, **caractérisée en ce que** les tensioactifs non ioniques sont choisis parmi les alcools, les alpha-diols, les alkyl(C₁-C₂₀)phénols ou les acides gras polyéthoxylés, polypropoxylés ou polyglycérolés, ayant une chaîne grasse, le nombre de groupements oxyde d'éthylène ou oxyde de propylène pouvant aller notamment de 2 à 50 et le nombre de groupements glycérol pouvant aller de 2 à 30 ; les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines ou les oxydes de N-acyl(C₁₀-C₁₄)aminopropylmorpholine.

19. Composition selon la revendication 18, **caractérisée en ce que** les tensioactifs non ioniques sont choisis parmi les alkyl(C₆-C₂₄)polyglycosides.

20. Composition selon la revendication 16 ou 17, **caractérisée en ce que** les tensioactifs amphotères sont choisis parmi les alkyl(C₈-C₂₀)bétaïnes, les alkyl(C₈-C₂₀)amidoalkyl(C₆-C₈)bétaïnes, les alkylamphodiacétates.

21. Composition selon la revendication 16, **caractérisée en ce que** les tensioactifs cationiques sont choisis parmi les sels d'alkylamidoalkyltrialkylammonium, les dérivés d'imidazoline, ou les oxydes d'amines à caractère cationique.

22. Composition selon l'une quelconque des revendications 16 à 21, **caractérisée en ce que** les tensioactifs différents des tensioactifs anioniques sont présents en une quantité comprise entre 0,5 et 25 % en poids, de préférence entre 0,5 et 15 % en poids par rapport au poids total de la composition.

23. Composition selon l'une quelconque des revendications précédentes, **caractérisée en ce que** le milieu aqueux cosmétiquement acceptable comprend l'eau ou un mélange d'eau et d'un solvant cosmétiquement acceptable.

24. Composition selon la revendication 23, **caractérisée en ce que** le solvant cosmétiquement acceptable est choisi parmi les alcools inférieurs en C₁-C₄, les polyols et leurs mélanges.

25. Composition selon l'une quelconque des revendications précédentes, **caractérisée en ce qu'**elle comprend en outre un ou plusieurs additifs classiques choisis parmi des agents tensioactifs cationiques, des polymères anioniques, cationiques, non-ioniques amphotères ou zwitteroniques, des filtres UV, des parfums, des colorants, des épaississants naturels ou synthétiques, des alcools gras en C₁₂-C₃₀, des agents nacrants, des conservateurs, des agents de stabilisation de pH, des vitamines, des provitamines, des agents antimicrobiens, les agents anti-oxydants ou réducteurs, et les agents acides ou alcalins.

26. Composition selon la revendication 25, **caractérisée en ce que** les polymères cationiques sont choisis parmi les dérivés d'éther de cellulose quaternaires, les gommes de guar cationiques, les cyclopolymères cationiques, les polymères quaternaires de vinylpyrrolidone et de vinylimidazole, et leurs mélanges.

27. Composition selon l'une quelconque des revendications précédentes, **caractérisée en ce qu'**elle se présente sous la forme d'une émulsion huile-dans-eau.

28. Utilisation de la composition selon l'une quelconque des revendications précédentes, comme shampoing.

29. Procédé de traitement capillaire des cheveux consistant à appliquer sur les cheveux, une quantité efficace d'une composition telle que décrite à l'une quelconque des revendications 1 à 26, et à rincer après un éventuel temps de pose.
